(19) Europäisches Patentamt
European Patent Office
Office européen des brevets

(11) EP 2 923 690 B1

(12) FASCICULE DE BREVET EUROPEEN

(45) Date de publication et mention de la délivrance du brevet:
**30.10.2019 Bulletin 2019/44**

(21) Numéro de dépôt: **15161540.8**

(22) Date de dépôt: **27.03.2015**

(51) Int Cl.:
***A61Q 17/04*** (2006.01) ***A61Q 13/00*** (2006.01)
***A61K 8/35*** (2006.01) ***A61K 8/37*** (2006.01)

(54) **COMPOSITION POUR LA STABILISATION DE COMPOSÉS PHOTOSENSIBLES**

ZUSAMMENSETZUNG FÜR DIE STABILISIERUNG VON LICHTEMPFINDLICHEN VERBINDUNGEN

COMPOSITION FOR STABILISING PHOTOSENSITIVE COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.03.2014 FR 1452757**

(43) Date de publication de la demande:
**30.09.2015 Bulletin 2015/40**

(73) Titulaire: **Sensient Cosmetic Technologies**
**95130 Saint Ouen L'Aumone (FR)**

(72) Inventeurs:
- **Blain, Lucile**
**75002 PARIS (FR)**
- **Geneve, Christine**
**78570 ANDRESY (FR)**
- **Peltier, Marianne**
**78670 VILLENNES SUR SEINE (FR)**
- **Boulier, Virginie**
**95450 CONDECOURT (FR)**

(74) Mandataire: **Lavoix**
**2, place d'Estienne d'Orves**
**75441 Paris Cedex 09 (FR)**

(56) Documents cités:
**US-A1- 2003 108 492 US-A1- 2008 131 381**
**US-A1- 2010 254 923 US-A1- 2012 039 827**
**US-A1- 2013 034 511 US-B2- 7 166 273**

- **CHAUDHURI R K ET AL: "Design of a Photostabilizer Having Built-in Antioxidant Functionality and Its Utility in Obtaining Broad-spectrum Sunscreen Formulations", PHOTOCHEMISTRY AND PHOTOBIOLOGY, WILEY-BLACKWELL PUBLISHING, INC, US, vol. 82, 1 janvier 2006 (2006-01-01), pages 823-828, XP002509174, ISSN: 0031-8655, DOI: 10.1562/2005-07-15-RA-612**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet de nouvelles compositions permettant de stabiliser des composés photosensibles, thermosensibles et/ou sensibles à l'oxydation, contre les effets des rayonnements ultraviolets.

La présente invention a également pour objet l'utilisation desdites compositions pour stabiliser notamment la coloration et/ou les parfums d'une formulation, telle qu'une formulation cosmétique.

Il est connu que les rayons UV (longueurs d'onde de 100 à 400 nm) peuvent initier des réactions radicalaires en chaîne qui entrainent la décomposition de molécules photosensibles. La majorité des produits cosmétiques est formulée avec des composés sensibles aux rayons UV, en particulier les colorants et les parfums. En effet, une partie des rayons UV-B (290 à 320 nm) et les rayons UV-A (320 à 400 nm) peuvent affecter l'intégrité de ces molécules photosensibles. Les produits conditionnés dans des emballages transparents ou semi-transparents sont particulièrement sujets à une exposition aux rayons UV. Les emballages standards non traités (polyéthylène, polypropylène, polytéréphtalate d'éthylène, verre, ...) protègent les formules au mieux des rayons UV-B. Il est donc particulièrement important d'assurer une protection de ces produits contre les UV-A.

Il existe des emballages spécifiques assurant une protection plus efficace, notamment des emballages en polyéthylène, polytéréphtalate d'éthylène, polypropylène ou polychlorure de vinyle, traités anti-UV, mais leur coût est très élevé, et des phénomènes de transfert des filtres UV de l'emballage vers les formules sont connus.

Une solution plus économique consiste à ajouter à la formule cosmétique des absorbeurs UV. Ces composés doivent cependant être choisis avec attention afin d'assurer une protection sur l'ensemble du spectre UV, et particulièrement celui des UV-A. Par ailleurs, certains filtres UV tel que l'avobenzone, sont eux-mêmes photosensibles et doivent être stabilisés par d'autres absorbeurs UV. Enfin, ces absorbeurs UV doivent également être choisis avec attention pour garantir le maintien des propriétés physico-chimiques des formules (telles que la couleur, l'odeur, la transparence, ...) dans lesquels ils sont incorporés et les stabiliser dans un temps suffisamment long pour assurer la durée de vie du produit.

US 7,150,876 décrit l'utilisation de diéthylhexyl syringylidènemalonate comme agent photo-stabilisateur de composés photosensibles, notamment de l'avobenzone. Toutefois, ce produit peut s'avérer coûteux.

US 2012/0039827 décrit des compositions solaires pouvant comprendre de l'éthyl-hexyl-salicylate (A), du butyl méthoxydibenzoylméthane (B) et du diéthylhexyl syringylidènemalonate.

La présente invention a donc pour but de fournir des compositions pour stabiliser des composés photosensibles, thermosensibles et/ou sensibles à l'oxydation, tels que des colorants, des parfums, des antioxydants ou des filtres ultraviolets, contre les effets des rayonnements ultraviolets, et ce à coût réduit.

Un des buts de l'invention est notamment de fournir de nouvelles compositions pour stabiliser la coloration et/ou les parfums contre les effets néfastes des rayonnements ultraviolets.

Un autre but de l'invention consiste en l'utilisation de nouvelles compositions pour stabiliser notamment la coloration et/ou les parfums d'une formulation telle qu'une formulation cosmétique.

Les inventeurs ont avantageusement montré que la composition stabilisante selon l'invention permet de stabiliser la coloration et/ou les parfums d'une formulation dans laquelle elle a été ajoutée. Une meilleure stabilisation a notamment été observée avec la composition selon l'invention comprenant l'association de trois absorbeurs UV (également appelé absorbeur ultraviolet), par rapport à des compositions comprenant uniquement un seul ou deux absorbeurs UV.

Ces stabilisations de la couleur et/ou des parfums ont avantageusement été obtenues avec une quantité de l'ordre de 0,3% seulement de la composition selon l'invention, ce qui permet un compromis coût/efficacité intéressant.

Les compositions selon l'invention permettent avantageusement d'éviter le vieillissement prématuré de formulations cosmétiques exposées à la lumière naturelle ou artificielle, à la chaleur ou à l'air, pouvant entraîner des phénomènes d'oxydation, de réduction, de catalyse... Ces compositions selon l'invention permettent donc en particulier de protéger des formulations cosmétiques conditionnées dans des emballages transparents.

*Composition*

La présente invention concerne une composition pour la stabilisation d'au moins un composé photosensible choisi parmi les colorants, les parfums, les filtres ultraviolets ou les antioxydants, contre les effets des rayonnements ultraviolets, ladite composition comprenant une association de :

- au moins un absorbeur ultraviolet (A) qui est l'éthyl-hexyl-salicylate ;
    - au moins un absorbeur ultraviolet (B) qui est le butyl méthoxydibenzoylméthane ;
    - au moins un absorbeur ultraviolet (C) qui est le diéthylhexyl syringylidènemalonate (C) ;

dans des proportions :

- de 70% à 75% en poids d'absorbeur ultraviolet (A), par rapport au poids total des absorbeurs ultraviolets (A), (B) et (C);
- de 15% à 20% en poids d'absorbeur ultraviolet (B), par rapport au poids total des absorbeurs ultraviolets (A), (B) et (C); et
- de 9% à 13% en poids d'absorbeur ultraviolet (C), par rapport au poids total des absorbeurs ultraviolets (A), (B) et (C).

Dans le cadre de l'invention, et sauf mention contraire, on entend par « absorbeur ultraviolet » (ou absorbeur UV), un composé chimique capable d'absorber des rayonnements ultraviolets dans une gamme comprise entre 4 et 400 nm, en particulier entre 200 et 400 nm, et tout particulièrement entre 290 et 400 nm (UV-A et UV-B).
Dans le cadre de l'invention, et sauf mention contraire, on entend par « composé photosensible », un composé pouvant être dégradé chimiquement suite à une exposition à des rayonnements UV émanant de la lumière naturelle ou artificielle.
Dans le cadre de l'invention, et sauf mention contraire, on entend par « composition comprenant au moins un absorbeur UV », une composition comprenant un absorbeur UV ou un mélange d'absorbeurs UV distincts.
Le 4-(ter.-butyl)-4'-méthoxy dibenzoylméthane (aussi dénommé butyl-méthoxydibenzoylméthane ou Avobenzone), répond à la formule suivante :

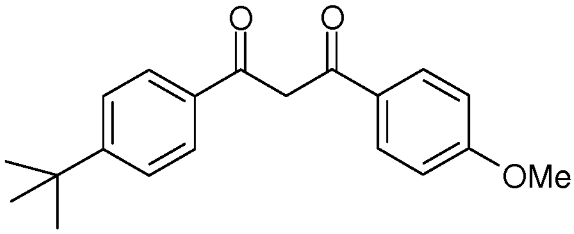

Le diéthylhexyl syringylidènemalonate a la formule suivante:

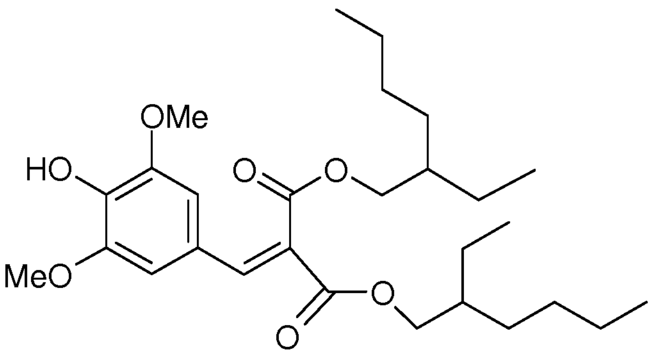

*Gammes et ratios*

La composition telle que définie précédemment comprend :

- de 70% à 75% en poids d'absorbeur ultraviolet (A), par rapport au poids total des absorbeurs ultraviolets (A), (B) et (C);
- de 15% à 20% en poids d'absorbeur ultraviolet (B), par rapport au poids total des absorbeurs ultraviolets (A), (B) et (C); et
- de 9% à 13% en poids d'absorbeur ultraviolet (C), par rapport au poids total des absorbeurs ultraviolets (A), (B) et (C).

Selon un mode de réalisation, la composition telle que définie précédemment comprend :

- de 71 % à 73% en poids d'absorbeur ultraviolet (A), par rapport au poids total des absorbeurs ultraviolets (A), (B) et (C);
- de 15% à 19% en poids d'absorbeur ultraviolet (B), par rapport au poids total des absorbeurs ultraviolets (A), (B) et (C); et
- de 10% à 13% en poids d'absorbeur ultraviolet (C), par rapport au poids total des absorbeurs ultraviolets (A), (B) et (C).

Selon un autre mode de réalisation, la composition telle que définie précédemment comprend :

- 71,5% en poids d'absorbeur ultraviolet (A), par rapport au poids total des absorbeurs ultraviolets (A), (B) et (C);
- 17% en poids d'absorbeur ultraviolet (B), par rapport au poids total des absorbeurs ultraviolets (A), (B) et (C); et
- 11,5 % en poids d'absorbeur ultraviolet (C), par rapport au poids total des absorbeurs ultraviolets (A), (B) et (C).

*Autres composants possibles*

Selon un mode de réalisation, la composition selon l'invention comprend en outre au moins un composé antioxydant choisi dans le groupe constitué : des thiols et dérivés ; des phénols, polyphénols et dérivés, tels que les flavonoïdes, les acides phénoliques, les tanins, les anthocyanes ; du β-carotène (provitamines A) ; des tocophérols (vitamine E) ou de ses esters comme l'alpha-tocophérol, le gamma-tocophérol, le delta-tocophérol.
Selon un mode de réalisation, le ratio entre au moins un composé antioxydant et la composition de l'invention est compris de 1 : 15 à 1 : 1.
Selon un mode de réalisation, la composition selon l'invention comprend en outre au moins un composé aux propriétés chélatantes choisi dans le groupe constitué de : l'acide ascorbique et ses esters comme l'ascorbate de sodium ou de calcium ; l'acide diacétyl 5-6-1 -ascorbique, l'acide palmityl 6-1-ascorbique, l'acide citrique et les citrates comme ceux de sodium, de potassium et de calcium ; l'acide tartrique et les tartrates comme ceux de sodium, potassium ; l'acide phytique et ses sels ; les acides diaminotétracarboxyliques tel que l'acide édétique ; le butylhydroxyanisol et butylhydroxytoluol ; les gallates d'octyle ou de dodécyle; les lactates de sodium, de potassium ou de calcium ; les lécithines ; le glutathion, les enzymes comme la catalase, les superoxydes dismutases et certaines peroxydases.
Selon un mode de réalisation, le ratio entre au moins un composé aux propriétés chélatantes et la composition de l'invention est compris de 1 : 15 à 1 : 6.
Selon un mode de réalisation, la composition selon l'invention comprend en outre au moins un tensioactif, synthétique ou biosourcé, choisi dans le groupe constitué des : tensioactifs anioniques, tels que les sels d'acides carboxyliques, les dérivés sulfonés, les dérivés sulfatés et dérivés phosphatés ; tensioactifs amphotères, tels que les bétaïnes, les dérivés d'imidazoline, les polypeptides ; et tensioactifs non-ioniques, tels que les non-ioniques à liaison ester, liaison éther ou liaison amide.
Selon un mode de réalisation, le ratio entre la composition de l'invention et au moins un tensioactif est compris de 1 : 1 à 1 : 20.
Selon un mode de réalisation, la composition selon l'invention comprend en outre au moins un solvant choisi dans le groupe constitué des : hydrocarbures, notamment les hydrocarbures aliphatiques ou aromatiques ou les hydrocarbures halogénés, alcools, glycols, cétones, esters, éthers et des éthers de glycol.
Selon un mode de réalisation, le ratio entre la composition stabilisante et au moins un solvant est compris de 1 : 1 à 1 : 20.
Selon un mode de réalisation, la composition selon l'invention se présente sous forme liquide.

*Utilisation*

La présente invention concerne également l'utilisation d'une composition, telle que décrite précédemment, pour stabiliser des composés photosensibles, thermosensibles et/ou sensibles à l'oxydation, tels que des colorants, des parfums, des antioxydants ou des filtres ultraviolets, des effets des rayonnements ultraviolets.
Selon l'invention, parmi les composés photosensibles, on peut citer les filtres ultraviolets, tels que : l'acide 4-amino-benzoïque (PABA), le méthosulfate camphre de benzalkonium, l'homosalate, le 2-Hydroxy-4-methoxybenzophenone (benzophenone-3), l'acide phénylbenzimidazole sulfonique, l'acide téréphtalylidène dicamphre sulfonique, le méthoxydibenzoylméthane de butyle, l'acide benzylidène camphre sulfonique, l'octocrylène, le polyacrylamidométhyl benzylidène camphre, l'éthylhexyl méthoxycinnamate, le PEG-25 de l'acide 4-amino-benzoïque, l'isoamyl p-méthoxycinnamate, l'éthylhexyl triazone, le drométrizole trisiloxane, le diéthylhexyl-butamido-triazone, le 4-méthylbenzylidène camphre, le 3-benzylidène camphre, l'éthylhexyl salicylate, l'éthylhexyl diméthyl PABA, l'acide 5-Benzoyl-4-hydroxy-2-méthoxybenzènesulfonique (Benzophenone-4), le sel de sodium de l'acide 2-hydroxy-4-méthoxybenzophenone-5-sulfonique (Benzophenone-5), le bis-benzotriazolyl tétraméthylbutylphénol de méthylène, le tétrasulfonate de disodium du phényl-dibenzimidazole, la bis-éthylhexyloxyphénol méthoxyphényl triazine, le polysilicone-15 ou le diéthylamino-hydroxybenzoyl-hexyl benzoate
Selon un mode de réalisation, les composés photosensibles sont des colorants, des parfums, des antioxydants ou des filtres ultraviolets.
Selon un mode de réalisation, la présente invention concerne l'utilisation d'une composition, telle que décrite précédemment, pour stabiliser la coloration d'une formulation des effets des rayonnements ultraviolets.
Selon un mode de réalisation, la présente invention concerne l'utilisation d'une composition, telle que décrite précédemment, pour stabiliser les colorants d'une formulation des effets des rayonnements ultraviolets. Selon un mode de réalisation, la composition selon l'invention permet d'éviter la dégradation de la coloration d'une formulation par les

rayonnements ultraviolets.

Selon un mode de réalisation, la présente invention concerne l'utilisation d'une composition, telle que décrite précédemment, pour stabiliser les parfums d'une formulation, des effets des rayonnements ultraviolets. Selon un mode de réalisation, la composition selon l'invention permet d'éviter la dégradation des parfums d'une formulation par les rayonnements ultraviolets.

A titre d'exemples d'ingrédient de parfums, on peut citer ceux compris dans la liste EPA Fragrance Ingredient List du 30 mars 2012 (http://www.epa.gov/opprd001/inerts/fmaingredient.pdf).

A titre d'exemples de colorants, on peut citer les colorants organiques ou les colorants naturels, tels que la garance, le carmin soluble, le caramel, la chlorophylle ou le cucurmin.

Plus particulièrement, à titre de colorants, on peut citer : Acid Black 1, Acid Black 52, Acid Blue 3, Acid Blue 9, Acid Blue 9 Ammonium Salt, Acid Blue 62, Acid Blue 74, Acid Green 1, Acid Green 25, Acid Green 50, Acid Orange 6, Acid Orange 7, Acid Red 18, Acid Red 27, Acid Red 33, Acid Red 51, Acid Red 73, Acid Red 87, Acid Red 92, Acid Red 95, Acid Red 184, Acid Red 195, Acid Violet 9, Acid Violet 43, Acid Yellow 3, Acid Yellow 23, Acid Yellow 73, Basic Blue 6, Acid Yellow 73 Sodium Salt, Aka2, Aka3, Aka102, Aka104(1), Aka105(1), Aka106, Aka201, Aka202, Aka203, Aka204, Aka205, Aka206, Aka207, Aka208, Aka213, Aka214, Aka215, Aka218, Aka219, Aka220, Aka221, Aka22, Aka225, Aka226, Aka227, Aka228, Aka230(1), Aka230(2), Aka231, Aka232, Aka401, Aka404, Aka405, Aka501, Aka502, Aka503, Aka504, Aka505, Annatto, Anthocyanins, Ao1, Ao2, Ao201, Ao202, Ao203, Ao204, Ao205, Ao403, Ao404, Astaxanthin, Basic Blue 6, Basic Blue 41, Basic Yellow 11, Beetroot, Blue 1,Blue 4, Brilliant Black 1, Bromocresol Green, Bromothymol Blue, Brown 1, Capsanthin/Capsorubin, Caramel, Carmine, Beta-Carotene, Carotenolds, Chlorophyllin-Copper Complex, CI 10006, CI 10020, CI 10316, CI 11680, CI 11710, CI 11725, CI 11920, CI 12010, CI 12085, CI 12120, CI 12150, CI 12370, CI 12420, CI 12480, CI 12490, CI 12700, CI 13015, CI 14270, CI 14700, CI 14720, CI 14815, CI 15510, CI 15525, CI 15580, CI 15620, CI 15630, CI 15800, CI 15850, CI 15865, CI 15880, CI 15980, CI 15985, CI 16035, CI 16185, CI 16230, CI 16255, CI 16290, CI 17200, CI 18050, CI 18130, CI 18690, CI 18736, CI 18820, CI 18965, CI 19140, CI 20040, CI 20170, CI 20470, CI 21100, CI 21108, CI 21230, CI 24790, CI 26100, CI 27290, CI 27755, CI 28440, CI 40215, CI 40800, CI 40820, CI 40825, CI 40850, CI 42045, CI 42051, CI 42053, CI 42080, CI 42090, CI 42100, CI 42170, CI 42510, CI 42520, CI 42735, CI 44045, CI 44090, CI 45100, CI 45190, CI 45220, CI 45350, CI 45370, CI 45380, CI 45396, CI 45405, CI 45410, CI 45425, CI 45430, CI 47000, CI 47005, CI 50325, CI 50420, CI 51319, CI 58000, CI 59040, CI 60724, CI 60725, CI 60730, CI 61565, CI 61570, CI 61585, CI 62045, CI 69800, CI 69825, CI 71105, CI 73000, CI 73015, CI 73360, CI 73385, CI 73900, CI 73915, CI 74100, CI 74160, CI 74180, CI 74260, CI 75100, CI 75120, CI 75125, CI 75130, CI 75135, CI 75170, CI 75300, CI 75470, CI 75810, CI 77000, CI 77002, CI 77004, CI 77007, CI 77015, CI 77120, CI 77163, CI 77220, CI 77231, CI 77267, CI 77268:1, CI 77288, CI 77289, CI 77346, CI 77400, CI 77480, CI 77713, CI 77742, CI 77745, CI 77820,CI 77947, Cochineal, Crocus Sativus Flower Extract, Curry Red, Daidai201, Daidai203, Daidai204, Daidai205, Daidai206, Daidai207, Daidai401, Daidai402, Daidai403, Dihydroxyacetone, Direct BLue 86, Dunaliella Bardawil Powder, Erythrulose, Ext. Violet 2, Ext. Yellow 7, Ext. Yellow 7 Lake, Fast Green FCF, Fluorescent Brightener 230, Fluorescent Brightener 236, Gardenia Florida Extract, Green 3, Green 5, Green 6, Green 8, Haematococcus Pluvialis Powder, Haematoxylon Campechianum Wood Extract, Henna, Katsu201, Ki4, Ki5, Ki201, Ki202(1), Ki202(2), Ki203, Ki204, Ki205, Ki401, Ki402, Ki403(1), Ki404, Ki405, Ki406, Ki407, Kuro401, Lactoflavin, Lawsone, Midori3, Midori201, Midori202, Midori204,k Midori205, Midori401, Midori402, Murasaki201, Murasaki401, Natural Red 26, Ninhydrin, Orange 4, Orange 5, Orange 10, Orange 11, Ponceau SX, Pyrophyllite, Red 4, Red 17, Red 21, Red 22, Red 27, Red 28, Red 30, Red 31,Red 33, Red 34,Red 36, Red 40, Solvent Green 3, Solvent Green 7, Solvent Orange 1, Solvent Red 1, Solvent Red 3, Solvent Red 23, Solvent Red 24, Solvent Red 43, Solvent Red 48, Solvent Red 49:1, Solvent Red 72, Solvent Red 73, Solvent Violet 13, Solvent Yellow 18, Solvent Yellow 29, Solvent Yellow 33, Solvent Yellow 44, Sunset Yellow, Vat Red 1, Violet 2, Yellow 5, Yellow 6, Yellow 7, Yellow 8, Yellow 10, Yellow 11.

Selon un mode préféré, à titre de colorants, on peut citer le Blue 1 (CI 42090), le Red 33 (CI 17200), le Red 4 (CI 14700) et le Ext. Violet 2 (CI 60730).

En particulier, la présente invention concerne l'utilisation d'une composition, telle que décrite précédemment, pour stabiliser les colorants ou les parfums dans tous types de formulations cosmétiques, plus particulièrement des produits de soin ou d'hygiène, des parfums ou eaux de toilette. De préférence, les formulations cosmétiques sont des shampooings, des gels douche, des bains de bouche, des lotions, des sérums, ou des gels.

Afin de préserver la coloration des effets des rayons UV, on ajoute la composition stabilisante susmentionnée à une formulation en une quantité appropriée. Cette quantité dépendra de la nature des colorants, de la nature du parfum, de la présence de composés pouvant entraîner des phénomènes d'oxydation, de réduction, de catalyse et de la présence de divers composés photosensibles, mais aussi du degré d'exposition de la formulation aux rayons UV. Elle sera donc notamment fonction de la transparence du récipient aux rayons UV et de la durée de vie de la formulation.

Selon un mode de réalisation, la composition selon l'invention est introduite dans une formulation en une teneur comprise de 0,01% à 3%, de préférence de 0,05% à 2%, et plus particulièrement de 0,1% à 1% en poids par rapport au poids total de la formulation.

La présente invention concerne également une méthode pour stabiliser des composés photosensibles, thermosensibles et/ou sensibles à l'oxydation, tels que les colorants, les parfums, les antioxydants ou les filtres ultraviolets, ladite méthode comprenant l'ajout d'une composition stabilisante telle que décrite précédemment dans une formulation comprenant lesdits composés photosensibles.

**Exemples**

*Fournisseurs*

- Ethylhexyl salicylate ; Merck, Ashland, Symrise, DSM
- Avobenzone ; Merck, Ashland, Symrise, DSM
- Diéthylhexyl syringylidène malonate :Merck
- Blue 1 (CI 42090) : Sensient Cosmetic Technologies
- Red 33 (CI 17200) : Sensient Cosmetic Technologies
- Red 4 (CI 14700) : Sensient Cosmetic Technologies
- Ext. Violet 2 (CI 60730) : Sensient Cosmetic Technologies
- Parfum I : Sensient Fragrances
- Parfum II : Sensient Fragrances
- Parfum III : Sensient Fragrances

*Colorants*

- $A_1$ : Blue 1 - CI 42090
- $B_1$: Red 33 - CI 17200
- $C_1$ : Red 4 - CI 14700
- $D_1$: Ext. Violet 2 - CI 60730

*Parfums*

- Parfum I : Note vanille (nom commercial : Vanilla)
- Parfum II : Notes balsamique, épicée, musc (nom commercial : Baby)
- Parfum III : Notes florale, vanille, ambrée (nom commercial : Blue)

Mesure indice ORAC :

Etude spectrofluorimétrie faite en mélangeant dans une cuvette du spectophotomètre l'échantillon avec de la fluorescéine diluée, à une température de 37°C. A t = 0, la source de radicaux AAPH (2,2'-azobis(2-amidinopropane) dihydrochloride) a été ajoutée au mélange. La mesure de la diminution de la fluorescence a été faite toutes les 100 secondes.

**Exemple 1 : Préparation des compositions stabilisantes**

L'éthylhexyl salicylate a été prémélangé avec le diéthylhexyl syringylidènemalonate, puis le mélange a été chauffé à 45°C. L'avobenzone a ensuite été incorporé dans le mélange à température de 45°C par petites fractions, sous agitation.
Les compositions suivantes ont été préparées :

| **Composition 1** (comparative) | **%** |
|---|---|
| Ethylhexyl Salicylate | 84,5 |
| Avobenzone | 8,5 |

(suite)

| **Composition 1** (comparative) | % |
|---|---|
| Diéthylhexyl Syringylidènemalonate | 7 |

| **Composition 2** (comparative) | % |
|---|---|
| Ethylhexyl Salicylate | 76 |
| Avobenzone | 17 |
| Diéthylhexyl Syringylidènemalonate | 7 |

| **Composition 3** (comparative) | % |
|---|---|
| Ethylhexyl Salicylate | 75,5 |
| Avobenzone | 8,5 |
| Diéthylhexyl Syringylidènemalonate | 16 |

| **Composition 4** (comparative) | % |
|---|---|
| Ethylhexyl Salicylate | 67 |
| Avobenzone | 17 |
| Diéthylhexyl Syringylidènemalonate | 16 |

| **Composition 5** (comparative) | % |
|---|---|
| Ethylhexyl Salicylate | 73,5 |
| Avobenzone | 14 |
| Diéthylhexyl Syringylidènemalonate | 11,5 |

| **Composition 6** (comparative) | % |
|---|---|
| Ethylhexyl Salicylate | 77 |
| Avobenzone | 11,5 |
| Diéthylhexyl Syringylidènemalonate | 11,5 |

| **Composition 7** | % |
|---|---|
| Ethylhexyl Salicylate | 71,5 |
| Avobenzone | 17 |
| Diéthylhexyl Syringylidènemalonate | 11,5 |

**Exemple 2 : Préparation des formulations de produit moussant**

Des formules de produit moussant ont été préparées avec 0,1% d'une solution aqueuse à 0,01% d'un colorant

$A_1$, $B_1$, $C_1$ ou $D_1$; 0,1% de parfum I, II ou III ; et 0,3 % d'une composition stabilisante choisie parmi l'une des compositions stabilisantes 1 à 7 ci-dessus.

Formule de produit moussant :

| Formulation | % en poids par rapport au poids total de la composition |
|---|---|
| **Phase A** | |
| Sodium Lauryl Ether Sulfate (Texapon® NSO IS) | 30 |
| Cocamidopropyl Bétaine (Dehyton® K COS) | 22,5 |
| **Phase B** | |
| Composition stabilisante | 0,3 |
| Conservateur | 0,3 |
| Parfum | 0,1 |
| **Phase C** | |
| Sodium Lauryl Ether Sulfate (Texapon® NSO IS) | 10 |
| Cocamidopropyl Betaine (Dehyton® K COS) | 7,5 |
| **Phase D** | |
| Eau | QSP 100 |
| **Phase E** | |
| Solution aqueuse de colorant à 0,01% | 0,1 |

Mode opératoire :

- les phases A, B et C ont été indépendamment les unes des autres, pré-mélangées ;
- la phase B a été incorporée dans la phase C, et le tout a été mélangé ;
- le mélange des phases B et C obtenu a été incorporé à son tour dans la phase A ;
- la phase D a été ajoutée lentement au mélange des phases A, B et C ;
- une fois que le mélange est homogène, la phase E a été ajoutée.

Plusieurs formulations ont été préparées en associant un parfum, un colorant et une composition stabilisante de la façon suivante :

- Parfum I + Colorant $A_1$, $B_1$, $C_1$ ou $D_1$ + Composition 1 / 2 / 3 / 4 / 5 / 6 ou 7
- Parfum II + Colorant $A_1$, $B_1$, $C_1$ ou $D_1$ + Composition 1 / 2 / 3 / 4 / 5 / 6 ou 7
- Parfum III + Colorant $A_1$, $B_1$, $C_1$ ou $D_1$ + Composition 1 / 2 / 3 / 4 / 5 / 6 ou 7

Chaque série a été préparée en 3 exemplaires et placée :

- à l'obscurité (témoins) ;
- dans une chambre de vieillissement accéléré aux UV (Suntest) pendant 24 et 48h ;
- à la lumière naturelle.

Le Suntest est un modèle CPS+ de la société Atlas / Ametek muni d'une lampe xénon de 1500 W équipée d'un filtre verre vitre permettant la simulation de la lumière naturelle du soleil derrière un vitrage de 3mm d'épaisseur. L'intensité d'éclairement a été fixée à son niveau maximal (60W/$m^2$).

Après passage au Suntest, la décoloration et la dégradation du parfum ont été évalués pour chacun des échantillons.

La décoloration a été évaluée à l'aide d'un spectrocolorimètre en transmission, modèle CM-5 de la société Konica Minolta. Les valeurs de $\Delta E$ des échantillons, après 24h et 48h d'exposition aux UV, ont été mesurées. La valeur de $\Delta E$ correspond à l'écart colorimétrique entre les échantillons et le témoin (n'ayant pas été exposé au Suntest) dans l'espace de couleur L*a*b*, suivant la formule suivante :

$$\Delta E^* = \sqrt{(L_1 - L_2)^2 + (a_1 - a_2)^2 + (b_1 - b_2)^2}$$

où :
$L_1$, $a_1$, $b_1$ sont les coordonnées dans l'espace colorimétrique L*a*b* de la première couleur à comparer (témoin) et $L_2$, $a_2$, $b_2$ celles de la seconde (échantillon)

Plus les valeurs de ΔE sont faibles, moins la coloration des formulations est dégradée.

Résultats obtenus pour les formulations préparées avec les compositions 2, 4 et 7 :

| Parfum | Colorant | Temps (h) | Témoin (ΔE) | Composition 2(ΔE) | Composition 4(ΔE) | Composition 7(ΔE) |
|---|---|---|---|---|---|---|
| Parfum I | $A_1$ | 24 | 47,23 | 3,9 | 3,65 | 2,73 |
| | | 48 | 47,04 | 13,33 | 4,58 | 3,42 |
| | $B_1$ | 24 | 53,92 | 13,36 | 10,97 | 8,4 |
| | | 48 | 53,66 | 29,46 | 21,38 | 21,93 |
| | $C_1$ | 24 | 33,78 | 1,95 | 2,68 | 2,3 |
| | | 48 | 33,79 | 3,38 | 4,39 | 3,68 |
| | $D_1$ | 24 | 25,22 | 2,9 | 3,74 | 2,98 |
| | | 48 | 24,66 | 15,67 | 10,56 | 11,45 |
| | | | | | | |
| Parfum II | $A_1$ | 24 | 47,59 | 1,93 | 2,6 | 2,46 |
| | | 48 | 46,91 | 34,61 | 21,61 | 10,95 |
| | $B_1$ | 24 | 53,7 | 13,51 | 11,1 | 10,81 |
| | | 48 | 53,45 | 47,09 | 52,33 | 23,29 |
| | $C_1$ | 24 | 34,22 | 1,56 | 1,77 | 2,07 |
| | | 48 | 34,21 | 3,51 | 3,52 | 3,64 |
| | $D_1$ | 24 | 26,81 | 1,95 | 2,44 | 2,9 |
| | | 48 | 25,56 | 19,76 | 16,31 | 9,69 |
| | | | | | | |
| Parfum III | $A_1$ | 24 | 48,77 | 2,19 | 2,47 | 2,29 |
| | | 48 | 48,7 | 23,72 | 3,93 | 3,97 |
| | $B_1$ | 24 | 54,99 | 13,19 | 10,63 | 9,93 |
| | | 48 | 54,91 | 53,03 | 19,01 | 20,4 |
| | $C_1$ | 24 | 33,99 | 1,45 | 1,57 | 1,57 |
| | | 48 | 33,84 | 4,09 | 3,1 | 2,7 |
| | $D_1$ | 24 | 23,95 | 2,33 | 2,22 | 2,22 |
| | | 48 | 23,49 | 18,34 | 6,59 | 7,56 |

**Exemple 3 comparatif : Comparaison de formulations de produit moussant**

Les compositions a, b et c suivantes ont été préparées :

| **Composition a** | **%** |
|---|---|
| Ethylhexyl Salicylate | 83 |

(suite)

| Composition a | % |
|---|---|
| Avobenzone | 17 |

| Composition b | % |
|---|---|
| Ethylhexyl Salicylate | 88,5 |
| Diéthylhexyl Syringylidènemalonate | 11,5 |

| Composition c | % |
|---|---|
| Diéthylhexyl Syringylidènemalonate | 100 |

Six formulations différentes ont été préparées avec ces compositions a, b et c, selon le mode opératoire de l'exemple 2, lesdites formulations comprenant le parfum II et les colorants B ou D.

Les compositions a et b ont été incorporées à 0,3% dans la formulation de produit moussant, c'est-à-dire dans les mêmes proportions que les compositions stabilisantes 2, 4 et 7. La composition c a été incorporée à 0,15% dans la formulation de produit moussant.

Résultats de ΔE obtenus pour les formulations préparées avec les compositions 2, 4, 7, a, b et c :

Colorant B :

| | Blanc (sans composition stabilisante de l'invention) | Composition | | | | | |
|---|---|---|---|---|---|---|---|
| | | 2 | 4 | 7 | a | b | c |
| 24h | 53,7 | 13,51 | 11,1 | 10,81 | 35,49 | 36,70 | 12,45 |
| 48h | 53,45 | 47,09 | 52,33 | 23,29 | 55,40 | 54,98 | 54,35 |

Colorant D :

| | Blanc (sans composition stabilisante de l'invention) | Composition | | | | | |
|---|---|---|---|---|---|---|---|
| | | 2 | 4 | 7 | a | b | c |
| 24h | 26,81 | 1,95 | 2,44 | 2,9 | 12,81 | 25,98 | 5,46 |
| 48h | 25,56 | 19,76 | 16,31 | 9,69 | 26,36 | 30,34 | 18,12 |

Ces résultats ont permis de montrer que les compositions stabilisantes selon l'invention, comprenant l'association éthylhexylsalicylate/avobenzone/diéthylhexyl syringylidènemalonate, permettent une meilleure stabilisation de la coloration par rapport à des compositions comprenant uniquement l'association de deux composés : éthylhexylsalicylate/avobenzone ou éthylhexylsalicylate/diéthylhexyl syringylidènemalonate.

La composition c a été incorporée à 0,15% dans la formule de produit moussant, ce qui est proche du pourcentage d'utilisation recommandé par le fournisseur du diéthylhexyl syringylidènemalonate (0,10%). Or, des résultats comparables, voire meilleurs, ont été obtenus, en termes de stabilisation de la coloration, avec les compositions stabilisantes de l'invention.

De ce fait, la quantité de diéthylhexyl syringylidènemalonate peut être réduite dans les compositions stabilisantes de l'invention pour une efficacité meilleure voire équivalente. Les compositions selon l'invention ont donc un rapport qualité/prix meilleur que la composition c.

**Exemple 4: Evaluation de la stabilisation des parfums**

Les formulations préparées avec les parfums I, II et III ont été évaluées olfactivement afin de déterminer l'impact de l'ajout d'une composition stabilisante sur des échantillons conservés à l'obscurité et après passage des échantillons

pendant 72h au Suntest.

Les échantillons ont été évalués comme suit :

- Pas de dénaturation du parfum +++
- Dénaturation faible du parfum ++
- Dénaturation moyenne du parfum +
- Dénaturation complète du parfum 0

Parfum I

| | Blanc (sans composition) | Composition 7 | Composition c |
|---|---|---|---|
| Obscurité | +++ | +++ | ++ |
| 72h suntest | + | ++ | 0 |

Parfum II

| | Blanc (sans composition) | Composition 7 | Composition c |
|---|---|---|---|
| Obscurité | +++ | +++ | ++ |
| 72h suntest | + | ++ | 0 |

Parfum III

| | Blanc (sans composition) | Composition 7 | Composition c |
|---|---|---|---|
| Obscurité | +++ | +++ | ++ |
| 72h suntest | + | ++ | 0 |

**Exemple 5: Effet synergique**

Les compositions c, d et e suivantes ont été préparées :

| **Composition c** | **%** |
|---|---|
| Diéthylhexyl Syringylidènemalonate | 100 |

| **Composition d** | **%** |
|---|---|
| Ethylhexyl Salicylate | 100 |

| **Composition e** | **%** |
|---|---|
| Avobenzone | 100 |

L'indice ORAC de la composition n°7 (exemple 1) et des compositions comparatives c, d, et e a été mesuré par spectro-fluorimétrie. Les résultats sont regroupés dans le tableau suivant :

| **Compositions** | **Indice ORAC ($\mu$mol Te/kg)** |
|---|---|
| Composition n°7 (exemple 1) | 102 240 |

(suite)

| Compositions | Indice ORAC (μmol Te/kg) |
|---|---|
| Composition c (comparative, composé (C) seul) | 7430 |
| Composition d (comparative, composé (A) seul) | 2013 |
| Composition e (comparative, composé (B) seul) | 13487 |

Les résultats montrent un effet synergique de la composition n°7 selon l'invention par rapport aux compositions c, d et e. En effet, l'indice ORAC de la composition n°7 (comprenant la combinaison de l'éthylhexylsalicylate, l'avobenzone et le diéthylhexyl syringylidènemalonate) de 102 240 μmol Te/kg va nettement au-delà de la somme des indices ORAC des compositions c, d et e :

$$71{,}5*2013/100 + 17*13487/100 + 11{,}5*7430/100 = 4586{,}5 < 102\ 240$$

L'indice ORAC permet de montrer le caractère antioxydant des compositions étudiées. Ainsi, la composition n°7 possède avantageusement un pouvoir antioxydant supérieur à la somme des pouvoirs oxydants des compositions c, d et e.

**Exemple 6**: **Etude comparative**

Les compositions f, g, h, i et j suivantes ont été préparées :

| Composition f | % |
|---|---|
| Ethylhexyl Salicylate | 90 |
| Avobenzone | 5 |
| Diéthylhexyl Syringylidènemalonate | 5 |

| Composition g | % |
|---|---|
| Ethylhexyl Salicylate | 65 |
| Avobenzone | 30 |
| Diéthylhexyl Syringylidènemalonate | 5 |

| Composition h | % |
|---|---|
| Ethylhexyl Salicylate | 70 |
| Avobenzone | 5 |
| Diéthylhexyl Syringylidènemalonate | 25 |

| Composition i | % |
|---|---|
| Ethylhexyl Salicylate | 56 |
| Avobenzone | 22 |
| Diéthylhexyl Syringylidènemalonate | 22 |
| **Composition j** | **%** |
| Homosalate | 60 |

(suite)

| Composition j | % |
|---|---|
| Avobenzone | 20 |
| Diéthylhexyl Syringylidènemalonate | 20 |

Ces compositions comparatives diffèrent des compositions selon l'invention en raison de la proportion des composés (A), (B) ou (C), et/ou de la nature du composé (A).

L'indice ORAC de la composition n°7 (exemple 1) et des compositions comparatives f, g, h, i et j a été mesuré. Les résultats sont regroupés dans le tableau suivant :

| Compositions | Indice ORAC (μmol Te/kg) |
|---|---|
| Composition n°7 (exemple 1) | 102 240 |
| Composition f (comparative) | < 2000 |
| Composition g (comparative) | < 2000 |
| Composition h (comparative) | 4810 |
| Composition i (comparative) | 3925 |
| Composition j (comparative) | 4270 |

Les résultats démontrent que la composition n°7 selon l'invention présente un indice ORAC très supérieur à celui des compositions comparatives f, g, h, i et j. Ainsi, la composition n°7 présente avantageusement un caractère antioxydant bien supérieur à celui des compositions f, g, h, i et j.

## Revendications

**1.** Composition pour la stabilisation d'au moins un composé photosensible choisi parmi les colorants, les parfums, les filtres ultraviolets ou les antioxydants, contre les effets des rayonnements ultraviolets, ladite composition comprenant une association d'éthyl-hexyl-salicylate (A), de butyl méthoxydibenzoylméthane (B) et de diéthylhexyl syringylidènemalonate (C) dans des proportions:

- de 70% à 75% en poids de (A), par rapport au poids total de (A), (B) et (C);
- de 15% à 20% en poids de (B), par rapport au poids total de (A), (B) et (C); et
- de 9% à 13% en poids de (C), par rapport au poids total de (A), (B) et (C).

**2.** Composition selon la revendication 1, comprenant :

- de 71 % à 73% en poids de (A), par rapport au poids total de (A), (B) et (C);
- de 15% à 19% en poids de (B), par rapport au poids total de (A), (B) et (C); et
- de 10% à 13% en poids de (C), par rapport au poids total de (A), (B) et (C).

**3.** Composition selon la revendication 2, comprenant

- 71,5% en poids de (A), par rapport au poids total de (A), (B) et (C);
- 17% en poids de (B), par rapport au poids total de (A), (B) et (C); et
- 11,5 % en poids de (C), par rapport au poids total de (A), (B) et (C).

**4.** Utilisation d'une composition selon l'une quelconque des revendications 1 à 3, pour stabiliser des composés photosensibles, thermosensibles et/ou sensibles à l'oxydation, tels que des colorants, des parfums, des antioxydants ou des filtres ultraviolets des effets des rayonnements ultraviolets.

**5.** Utilisation selon la revendication 4, dans laquelle les composés photosensibles sont des colorants ou des parfums.

## Patentansprüche

1. Zusammensetzung zur Stabilisierung mindestens einer lichtempfindlichen Verbindung ausgewählt aus Farbstoffen, Parfums, UV-Filtern oder Antioxidantien gegen die Wirkung ultravioletter Strahlungen, wobei die Zusammensetzung eine Kombination von Ethylhexylsalicyalt (A), Butylmethoxydibenzoylmethan (B) und Diethylhexylsyringylidenmalonat (C) in den Verhältnissen:

   - von 70 bis 75 Gew.-% von (A), bezogen auf das Gesamtgewicht von (A), (B) und (C);
   - von 15 bis 20 Gew.-% von (B), bezogen auf das Gesamtgewicht von (A), (B) und (C); und
   - von 9 bis 13 Gew.-% von (C), bezogen auf das Gesamtgewicht von (A), (B) und (C) umfasst.

2. Zusammensetzung gemäß Anspruch 1, umfassend:

   - von 71 bis 73 Gew.-% von (A), bezogen auf das Gesamtgewicht von (A), (B) und (C);
   - von 15 bis 19 Gew.-% von (B), bezogen auf das Gesamtgewicht von (A), (B) und (C); und
   - von 10 bis 13 Gew.-% von (C), bezogen auf das Gesamtgewicht von (A), (B) und (C).

3. Zusammensetzung gemäß Anspruch 2, umfassend

   - 71,5 Gew.-% von (A), bezogen auf das Gesamtgewicht von (A), (B) und (C);
   - 17 Gew.-% von (B), bezogen auf das Gesamtgewicht von (A), (B) und (C); und
   - 11,5 Gew.-% von (C), bezogen auf das Gesamtgewicht von (A), (B) und (C).

4. Verwendung einer Zusammensetzung gemäß einem der Ansprüche 1 bis 3 zur Stabilisierung von lichtempfindlichen, temperaturempfindlichen und/oder oxidationsempfindlichen Verbindungen, wie Farbstoffen, Parfums, Antioxidantien oder UV-Filtern gegen die Wirkung ultravioletter Strahlungen.

5. Verwendung gemäß Anspruch 4, wobei die lichtempfindlichen Verbindungen Farbstoffe und Parfums sind.

## Claims

1. Composition for the stabilisation of at least one photosensitive compound chosen from colorants, perfumes, ultraviolet filters and antioxidants against the effects of ultraviolet radiation, said composition comprising an association of ethylhexyl salicylate (A), butyl methoxybenzoylmethane (B) and diethylhexyl syringylidenemalonate (C) in proportions of:

   - from 70% to 75% by weight of (A), based on the total weight of (A), (B) and (C);
   - from 15% to 20% by weight of (B), based on the total weight of (A), (B) and (C); and
   - from 9% to 13% by weight of (C), based on the total weight of (A), (B) and (C).

2. Composition according to claim 1, comprising:

   - from 71% to 73% by weight of (A), based on the total weight of (A), (B) and (C);
   - from 15% to 19% by weight of (B), based on the total weight of (A), (B) and (C); and
   - from 10% to 13% by weight of (C), based on the total weight of (A), (B) and (C).

3. Composition according to claim 2, comprising:

   - 71.5% by weight of (A), based on the total weight of (A), (B) and (C);
   - 17% by weight of (B), based on the total weight of (A), (B) and (C); and
   - 11.5% by weight of (C), based on the total weight of (A), (B) and (C).

4. Use of a composition according to any one of claims 1 to 3 for stabilising photosensitive, thermosensitive and/or oxidation-sensitive compounds, such as colorants, perfumes, antioxidants or ultraviolet filters, against the effects of ultraviolet radiation.

5. Use according to claim 4, wherein the photosensitive compounds are colorants or perfumes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 7150876 B
- US 20120039827 A